# EUROPEAN PATENT APPLICATION

(11) **EP 4 011 275 A1**
(43) Date of publication of application: **15.06.2022**
(21) Application number: 20849545.7
(22) Date of filing: 17.07.2020
(51) Int. Cl.: A61B 3/16

(54) **ULTRASONIC OPHTHALMIC TONOMETER**

(30) Priority: 05.08.2019 JP 2019143989
(71) Applicant: Nidek Co., Ltd., Gamagori-shi, Aichi, 443-0038 (JP)
(72) Inventor: UEMURA, Tsutomu, Gamagori-shi Aichi 443-0038 (JP); MIWA, Tetsuyuki, Gamagori-shi Aichi 443-0038 (JP); HAMAGUCHI,Koji, Gamagori-shi Aichi 443-0038 (JP); NAKAMURA, Kenji, Gamagori-shi Aichi 443-0038 (JP)
(74) Representative: Hoefer & Partner Patentanwälte mbB
(86) International application number: PCT/JP2020/027937
(87) International publication number: WO 2021/024765

(57) **Abstract**

A technical object is to provide an ultrasonic ophthalmic tonometer which enables to properly irradiate a subject eye with an ultrasonic wave. The ultrasonic ophthalmic tonometer measures an intraocular pressure of the subject eye with using the ultrasonic wave. The ultrasonic ophthalmic tonometer includes a Langevin-type transducer which irradiates the subject eye with the ultrasonic wave, and a holding shape portion for preventing the Langevin-type transducer from rotating with respect to a jig when the Langevin-type transducer is assembled. The holding shape portion is provided in at least one of positions which are mirror-symmetrical with respect to a plane perpendicular to a sound axis of the Langevin-type transducer and positions which are rotationally symmetrical around the sound axis.

## Description

### Technical Field

The present disclosure relates to an ultrasonic ophthalmic tonometer that measures an intraocular pressure of a subject eye with using an ultrasonic wave.

### Background Art

As a non-contact type ophthalmic tonometer, an air injection type ophthalmic tonometer has generally been used so far. The air injection type ophthalmic tonometer converts an air pressure in a predetermined deformed state into an intraocular pressure by detecting a deformed state of a cornea when air is injected into the cornea and the air pressure injected into the cornea.

In addition, as the non-contact type ophthalmic tonometer, an ultrasonic ophthalmic tonometer that measures the intraocular pressure with using an ultrasonic wave has been proposed (refer to Patent Literature 1). The ultrasonic ophthalmic tonometer disclosed in Patent Literature 1 converts a radiation pressure in a predetermined deformed state into the intraocular pressure by detecting a deformed state of the cornea when the ultrasonic wave is radiated to the cornea and the radiation pressure injected to the cornea.

### Citation List

### Patent Literature

Patent Literature 1: JP-A-H05-253190

### Summary of Invention

However, a device disclosed in the related art cannot properly irradiate a cornea of a subject eye with an ultrasonic wave. For example, according to the device disclosed in Patent Literature 1, the ultrasonic wave cannot be applied to the subject eye to such an extent that the cornea is actually flattened or depressed, and the subject eye cannot be properly irradiated with the ultrasonic wave.

In view of problems in the related art, a technical object of the present disclosure is to provide an ultrasonic ophthalmic tonometer which enables to properly irradiate a subject eye with an ultrasonic wave.

In order to achieve the above-described object, the present disclosure includes configurations as follows.

(1) There is provided an ultrasonic ophthalmic tonometer that measures an intraocular pressure of a subject eye with using an ultrasonic wave. The ultrasonic ophthalmic tonometer includes a Langevin-type transducer that irradiates the subject eye with the ultrasonic wave, and a holding shape portion for preventing the Langevin-type transducer from rotating with respect to a jig when the Langevin-type transducer is assembled. The holding shape portion is provided in at least one of positions which are mirror-symmetrical with respect to a plane perpendicular to a sound axis of the Langevin-type transducer and positions which are rotationally symmetrical around the sound axis.

### Brief Description of Drawings

[FIG. 1] FIG. 1 is an external view of an ultrasonic ophthalmic tonometer.
[FIG. 2] FIG. 2 is an internal configuration diagram of the ultrasonic ophthalmic tonometer.
[FIG. 3] FIG. 3 is a view illustrating a structure of an ultrasonic actuator.
[FIG. 4] FIG. 4 is an enlarged view illustrating a portion of the ultrasonic actuator.
[FIG. 5A] FIG. 5A is a schematic view for illustrating a holding shape portion.
[FIG. 5B] FIG. 5B is a schematic view for illustrating the holding shape portion.
[FIG. 5C] FIG. 5C is a schematic view for illustrating the holding shape portion.
[FIG. 6A] FIG. 6A is a view illustrating a modification example of the holding shape portion.
[FIG. 6B] FIG. 6B is a view illustrating a modification example of the holding shape portion.
[FIG. 6C] FIG. 6C is a view illustrating a modification example of the holding shape portion.
[FIG. 6D] FIG. 6D is a view illustrating a modification example of the holding shape portion.
[FIG. 6E] FIG. 6E is a view illustrating a modification example of the holding shape portion.
[FIG. 6F] FIG. 6F is a view illustrating a modification example of the holding shape portion.
[FIG. 6G] FIG. 6G is a view illustrating a modification example of the holding shape portion.
[FIG. 6H] FIG. 6H is a view illustrating a modification example of the holding shape portion.
[FIG. 7A] FIG. 7A is a view illustrating a modification example of the holding shape portion.
[FIG. 7B] FIG. 7B is a view illustrating a modification example of the holding shape portion.
[FIG. 8] FIG. 8 is a block diagram illustrating a control system of the ultrasonic ophthalmic tonometer.

### Description of Embodiments

### <Embodiment>

An embodiment of the present disclosure will be described. An ultrasonic ophthalmic tonometer (for example, an ultrasonic ophthalmic tonometer 1) of the present embodiment measures an intraocular pressure of a subject eye with using an ultrasonic wave in a non-contact manner. The ultrasonic ophthalmic tonometer includes a Langevin-type transducer (for example, an ultrasonic actuator 100) and a holding shape portion (for example, a holding shape portion 140). The Langevin-type transducer irradiates the subject eye with the ultrasonic wave. The holding shape portion prevents the Langevin-type transducer from rotating with respect to a jig, for example, when the Langevin-type transducer is assembled. In addition, the holding shape portion is provided in at least one of positions which are mirror-symmetrical with respect to a plane perpendicular to a sound axis of the Langevin-type transducer and positions which are rotationally symmetrical around the sound axis. The ultrasonic ophthalmic tonometer of the present embodiment includes the above-described configuration. Accordingly, for example, the Langevin-type transducer can be properly assembled, and the subject eye can be properly irradiated with the ultrasonic wave. For example, a sufficient pressure can be generated to deform a cornea in a predetermined manner.

The Langevin-type transducer may have a sonotrode (for example, a sonotrode 131), a back mass (for example, a back mass 132), and an ultrasonic element (for example, an ultrasonic element 110) pinched between the sonotrode and the back mass. In this case, the holding shape portion may be provided in at least one of the sonotrode and the back mass.

The holding shape portion may be at least one of a cut portion, a recessed portion, a protruded portion, and a rough surface portion. For example, the holding shape portion has a structure for strengthening a holding force of the jig when assembled. The holding shape portions are disposed at positions which are mirror-symmetrical or rotationally symmetrical not to hinder generation of the ultrasonic wave as much as possible.

For example, the holding shape portion may be integrally formed with the sonotrode or the back mass to form the sonotrode or the back mass in a polygonal shape or an elliptical shape.

The Langevin-type transducer may have an opening portion (for example, an opening portion 101). In this case, the holding shape portion 140 may be provided inside the opening portion.

### <Example>

Hereinafter, examples according to the present disclosure will be described. For example, the ultrasonic ophthalmic tonometer of the present example measures the intraocular pressure of the subject eye with using the ultrasonic wave in a non-contact manner. For example, the ultrasonic ophthalmic tonometer measures the intraocular pressure by optically or acoustically detecting a shape change or an oscillation of the subject eye when the subject eye is irradiated with the ultrasonic wave. For example, the ultrasonic ophthalmic tonometer continuously irradiates the cornea with a pulse wave or a burst wave, and calculates the intraocular pressure, based on output information of the ultrasonic wave when the cornea is deformed into a predetermined shape. For example, the output information is a sound pressure of the ultrasonic wave, an acoustic radiation pressure, an irradiation time (for example, an elapsed time after a trigger signal is input), or a frequency. For example, when the cornea of the subject eye is deformed, the sound pressure of the ultrasonic wave, the acoustic radiation pressure, or an acoustic flow is used.

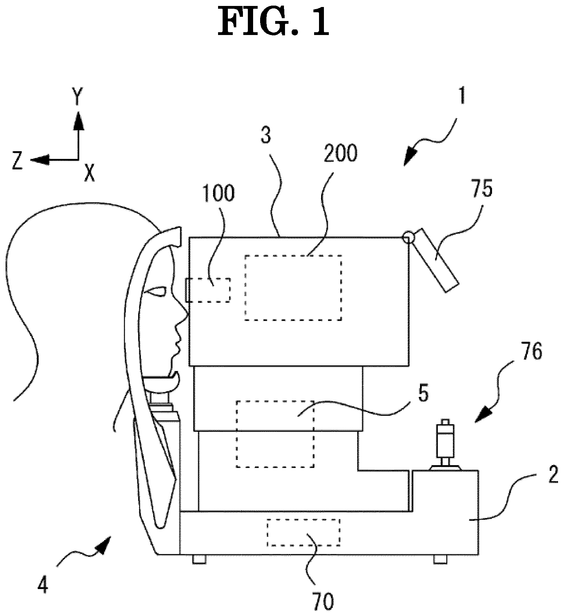

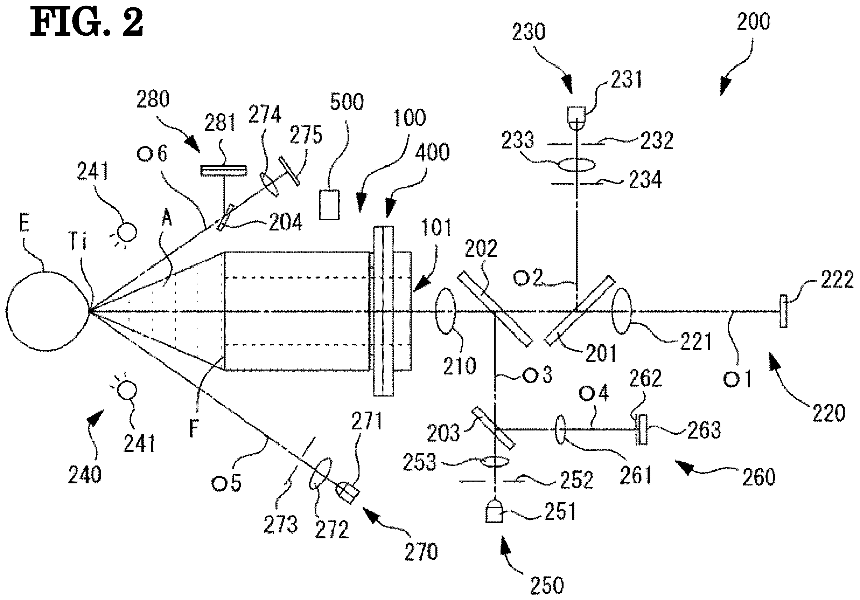

As illustrated in FIG. 1, for example, the ultrasonic ophthalmic tonometer 1 includes a base 2, a measurement unit 3, a face support unit 4, and a drive unit 5. the ultrasonic actuator 100 and the optical unit 200 (to be described later) are disposed inside the measurement unit 3. The face support unit 4 supports a face of the subject eye. For example, the face support unit 4 is installed on the base 2. For example, the drive unit 5 moves the measurement unit 3 with respect to the base 2 for alignment. The ultrasonic actuator 100 and the optical unit 200 are disposed inside the measurement unit 3 (refer to FIG. 2).

### <Ultrasonic Actuator>

For example, the ultrasonic actuator 100 irradiates a subject eye E with the ultrasonic wave. For example, the ultrasonic actuator 100 irradiates the cornea with the ultrasonic wave to generate the acoustic radiation pressure on the cornea. For example, the acoustic radiation pressure is a force acting in a traveling direction of a sound wave. For example, the ultrasonic ophthalmic tonometer 1 of the present example uses the acoustic radiation pressure to deform the cornea. The ultrasonic actuator 100 of the present example has a cylindrical shape, and an optical axis O1 of the optical unit 200 (to be described later) is disposed in the opening portion 101 at the center.

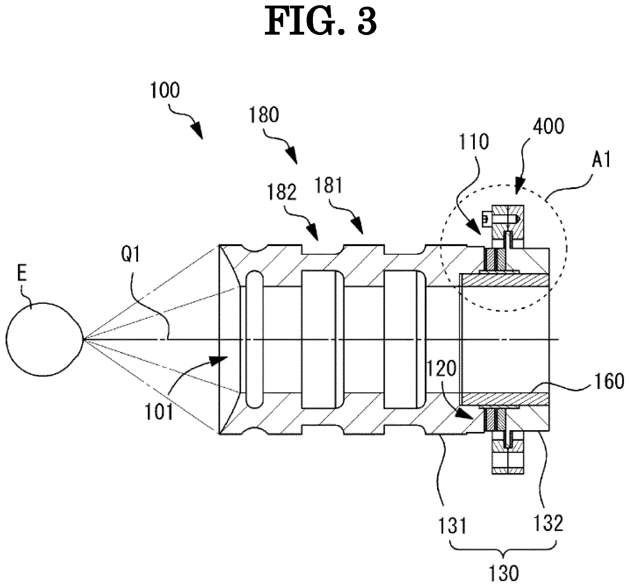

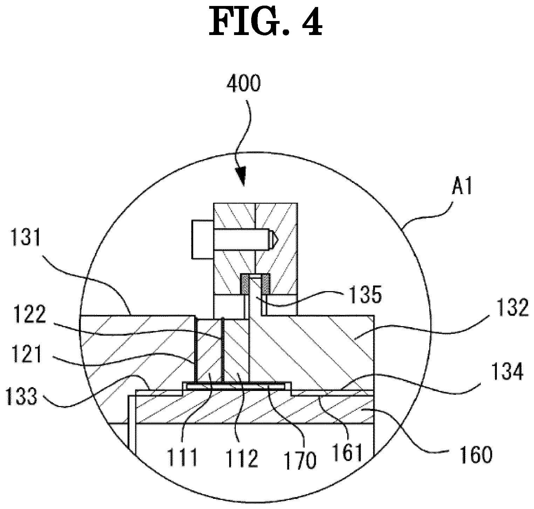

The ultrasonic actuator 100 of the present example is a so-called Langevin-type transducer. As illustrated in FIG. 3, for example, the ultrasonic actuator 100 includes an ultrasonic element 110, an electrode 120, a mass member 130, and a tightening member 160. The ultrasonic element 110 generates the ultrasonic wave. The ultrasonic element 110 may be a voltage element (for example, piezoelectric ceramics) or a magnetostrictive element. The ultrasonic element 110 of the present example has a ring shape. For example, the ultrasonic element 110 may be configured with a plurality of piezoelectric elements stacked. FIG. 4 is an enlarged view of a region A1 in FIG. 3. In the present example, as illustrated in FIG. 4, the ultrasonic element 110 includes two stacked piezoelectric elements (for example, a piezoelectric element 111 and a piezoelectric element 112). For example, electrodes 120 (electrode 121 and electrode 122) are respectively connected to the two piezoelectric elements. For example, the electrode 121 and the electrode 122 of the present example have the ring shape.

For example, the mass member 130 clamps the ultrasonic element 110. For example, the mass member 130 clamps the ultrasonic element 110 to increase tensile strength of the ultrasonic element 110 so that the ultrasonic element 110 can withstand a strong oscillation. In this manner, it is possible to generate a high-power ultrasonic wave. For example, the mass member 130 may be a metal block. For example, the mass member 130 includes the sonotrode (also referred to as a horn or a front mass) 131 and the back mass 132.

The sonotrode 131 is a mass member disposed in front of the ultrasonic element 110 (on the subject eye side). The sonotrode 131 propagates and amplifies the ultrasonic wave generated by the ultrasonic element 110. The sonotrode 131 of the present example has a hollow cylindrical shape (hollow columnar shape). A female screw portion 133 is partially formed on an inner circle side of the sonotrode 131. The female screw portion 133 is screwed to a male screw portion 161 formed in the tightening member 160 (to be described later).

The sonotrode 131 of the present example is a hollow cylinder having a non-uniform thickness. For example, the sonotrode 131 has a shape in which an outer diameter and an inner diameter are changed in a direction of a sound axis Q1 (longitudinal direction) of the hollow cylinder. For example, as illustrated in FIG. 3, the sonotrode 131 has an uneven portion 180 including a thick wall portion 181 and a thin wall portion 182. For example, the sound axis Q1 may be an axis parallel to a stacking direction of the ultrasonic element 110 and the electrode 120 of the Langevin-type transducer, or may be an axis parallel to an oscillation direction of the Langevin-type transducer.

The back mass 132 is a mass member disposed on a rear side of the ultrasonic element 110. The back mass 132 pinches the ultrasonic element 110 together with the sonotrode 131. For example, the back mass 132 has a cylindrical shape. A female screw portion 134 is partially formed in an inner circular portion of the back mass 132. The female screw portion 134 is screwed to the male screw portion 161 of the tightening member 160 (to be described later). In addition, the back mass 132 has flange portion 135. The flange portion 135 is held by a mounting portion 400.

For example, the tightening member 160 tightens the mass member 130 and the ultrasonic element 110 sandwiched between the mass members 130. For example, the tightening member 160 is a hollow bolt. For example, the tightening member 160 has a cylindrical shape, and includes the male screw portion 161 on the outer circular portion. The male screw portion 161 of the tightening member 160 is screwed to the female screw portions 133 and 134 formed inside the sonotrode 131 and the back mass 132. The sonotrode 131 and the back mass 132 are tightened in a direction in which both are closer to each other by the tightening member 160. In this manner, the ultrasonic element 110 sandwiched between the sonotrode 131 and the back mass 132 is tightened, and a pressure (clamp pressure) is applied. In the Langevin-type transducer, when the pressure increases, the sound pressure (or the acoustic radiation pressure) increases.

When the Langevin-type transducer is assembled, a side surface of the mass member 130 is supported by a jig (vice or clamp) to hold the Langevin-type transducer. However, when the mass member 130 has a perfect circular shape in a cross section perpendicular to the sound axis Q1, a holding force is insufficient when the jig holds the mass member 130. Therefore, when the sonotrode 131 and the back mass 132 are rotated in the direction in which both are closer to each other by the male screw portion 161, the sonotrode 131 or the back mass 132 slips with respect to the jig, and thus, a sufficient torque cannot be applied. In this manner, the pressure applied to the ultrasonic element 110 cannot be increased, and a desired sound pressure cannot be obtained.

Therefore, the Langevin-type transducer of the present example has the holding shape portion 140. The holding shape portion 140 is used for assembling the Langevin-type transducer. For example, the holding shape portion 140 has a shape for being held by a jig (vice, clamp, wrench, or socket) when the ultrasonic element 110 is tightened by the tightening member 160. For example, the holding shape portion 140 prevents the sonotrode 131 and the back mass 132 from slipping and rotating with respect to the jig. For example, the holding shape portion 140 is a cut portion (D-cut or H-cut), a recessed portion (groove or step), a protruded portion (protrusion or step), or a rough surface portion.

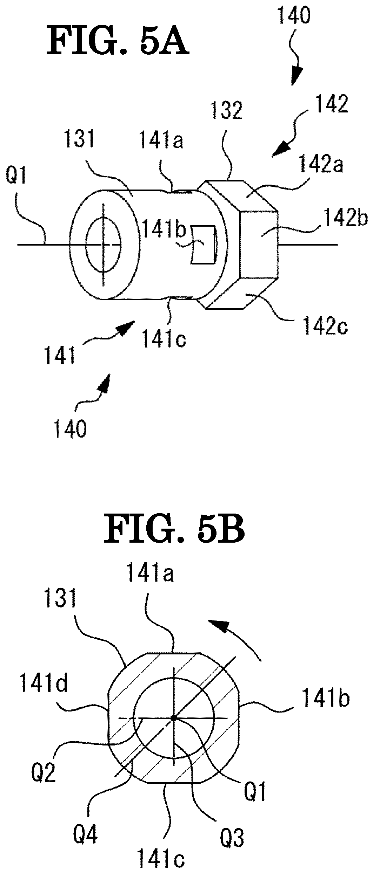

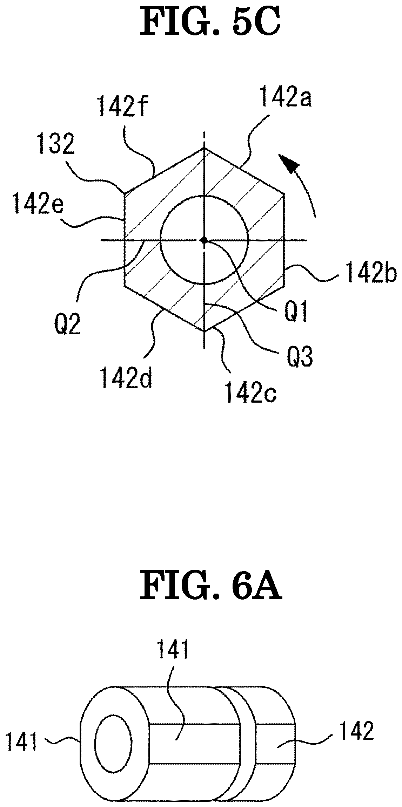

FIG. 5 is an example of the shape of the holding shape portion 140 of the present example. As illustrated in FIG. 5A, the holding shape portion 140 is provided in each of the sonotrode 131 and the back mass 132. For example, the sonotrode 131 has holding shape portions 141 having a shape in which a portion of the outer circular portion (side surface portion) is cut to be flat. For example, the holding shape portions 141 are provided at four sections of the sonotrode 131. FIG. 5B is a cross-sectional view when the sonotrode 131 is cut by a plane which is perpendicular to the sound axis Q1 and includes the holding shape portions 141. For example, the holding shape portions 141 are provided at positions which are rotationally symmetrical when the sonotrode 131 is rotated around the sound axis Q1. For example, when the sonotrode 131 is rotated 1/4 (90°) around the sound axis Q1, a position of a holding shape portion 141a is a position of a holding shape portion 141b, the position of the holding shape portion 141b is a position of a holding shape portion 141c, the position of the holding shape portion 141c is a position of a holding shape portion 141d, and the position of the holding shape portion 141d is the position of the holding shape portion 141a. In this way, the holding shape portions 141 are disposed at positions which are symmetrical four times. As a matter of course, without being limited to symmetry of four times, symmetry of n-times (n is a natural number of 2 or more) may be used.

In addition, the holding shape portions 141 are provided at positions which are mirror-symmetrical (line-symmetrical) with respect to an axis Q2 orthogonal to the sound axis Q1. In addition, the holding shape portions 141 are provided at positions which are mirror-symmetrical with respect to an axis Q3 orthogonal to the sound axis Q1 and the axis Q2. For example, as illustrated in FIG. 5B, the holding shape portion 141a and the holding shape portion 141c are provided at positions which are mirror-symmetrical with respect to the axis Q2. In addition, the holding shape portion 141b and the holding shape portion 141d are provided at positions which are mirror-symmetrical with respect to the axis Q3. Therefore, the holding shape portions 141 are provided at positions which are vertically and laterally symmetrical. In addition, with respect to an axis Q4 in an oblique direction, the holding shape portion 141a and the holding shape portion 141b are provided at positions which are mirror-symmetrical, and the holding shape portion 141c and the holding shape portion 141d are provided at positions which are mirror-symmetrical.

The back mass 132 has holding shape portions 142 having a shape in which an outer circular portion (side surface portion) is cut to be flat so that the whole back mass 132 has a hexagonal shape. FIG. 5C is a cross-sectional view when the back mass 132 is cut by a plane perpendicular to the sound axis Q1. The holding shape portions 142 are provided at six sections which are rotationally symmetrical when the back mass 132 is rotated around the sound axis Q1. For example, as illustrated in FIG. 5C, when viewed in a direction of the sound axis Q1, and when rotated 1/6 (60°) around the sound axis Q1, a position of a holding shape portion 142a is a position of a holding shape portion 142b, the position of the holding shape portion 142b is a position of a holding shape portion 142c, the position of the holding shape portion 142c is a position of a holding shape portion 142d, and the position of the holding shape portion 142d is a position of a holding shape portion 142e, the position of the holding shape portion 142e is a position of a holding shape portion 142f, and the position of the holding shape portion 142f is the position of the holding shape portion 142a. In this way, the holding shape portions 142 are disposed at positions which are symmetrical six times. As a matter of course, without being limited to symmetry of six times, symmetry of n-times (n is a natural number of 2 or more) may be used.

In addition, the holding shape portions 142 are provided at positions which are mirror-symmetrical with respect to the axis Q2 orthogonal to the sound axis Q1. In addition, the holding shape portions 142 are provided at positions which are mirror-symmetrical with respect to the axis Q3 orthogonal to the sound axis Q1 and the axis Q2. For example, as illustrated in FIG. 5C, the holding shape portion 142a and the holding shape portion 142c, and the holding shape portion 142d and the holding shape portion 142f are respectively provided at positions which are mirror-symmetrical with respect to the axis Q2. In addition, the holding shape portion 142a and the holding shape portion 142f, the holding shape portion 142b and the holding shape portion 142e, and the holding shape portion 142c and the holding shape portion 142d are respectively provided at positions which are mirror-symmetrical with respect to the axis Q3. In this way, the holding shape portions 140 are disposed at positions which are mirror-symmetrical with respect to a plane perpendicular to the sound axis Q1 of the Langevin-type transducer and positions which are rotationally symmetrical around the sound axis Q1.

As a matter of course, the holding shape portion 140 may be disposed in any one of positions which are mirror-symmetrical or positions which are rotationally symmetrical. For example, in FIG. 5B, the sonotrode 131 may have only the holding shape portion 141a and the holding shape portion 141b. In this case, the holding shape portion 140 is mirror-symmetrical with respect to the axis Q4, but is not rotationally symmetrical with respect to the sound axis Q1.

As illustrated in FIGs. 5A to 5C, the holding shape portions 141 of the sonotrode 131 and the holding shape portions 142 of the back mass 132 may be disposed in different shapes or at different positions, or may be disposed in the same shape or at the same position.

When the Langevin-type transducer is assembled, for example, a jig (vice, clamp, or spanner) is attached and fixed to at least two of the holding shape portions 141a to 141d provided at four sections of the sonotrode 131. Then, the jig (socket, wrench, or spanner) is attached to the holding shape portions 142 of the back mass 132, and the back mass 132 is rotated with respect to the sonotrode 131. In this manner, the sonotrode 131 and the back mass 132 are tightened by the tightening member 169. In this case, a reaction force is generated on a contact surface between the holding shape portion 140 and the jig. Accordingly, it is possible to prevent the sonotrode 131 or the back mass 132 from slipping and rotating with respect to the jig in a direction opposite to a tightening direction. In this manner, the sonotrode 131 and the back mass 132 can be properly tightened, and the ultrasonic element 110 can be sandwiched with a proper force.

In addition, a general-purpose socket can be used by forming the sonotrode 131 or the back mass 132 into a regular polygonal shape such as a regular hexagonal shape as the holding shape portion 140. In addition, the holding shape portion 140 can prevent the rotation of the mass member 130. Accordingly, the Langevin-type transducer is easily assembled with a predetermined torque by using a torque wrench.

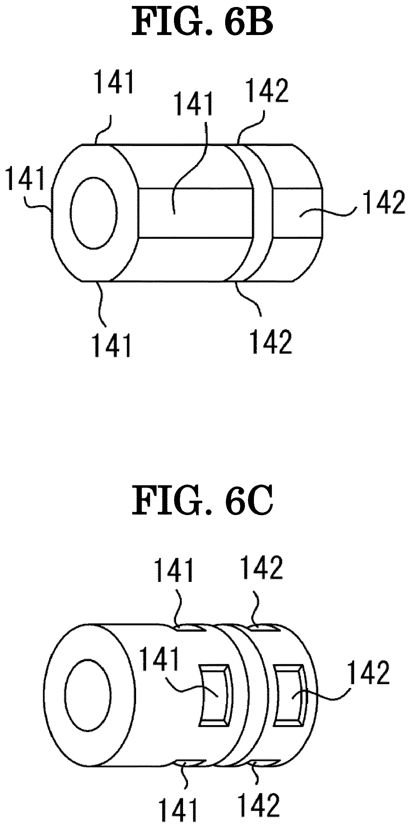

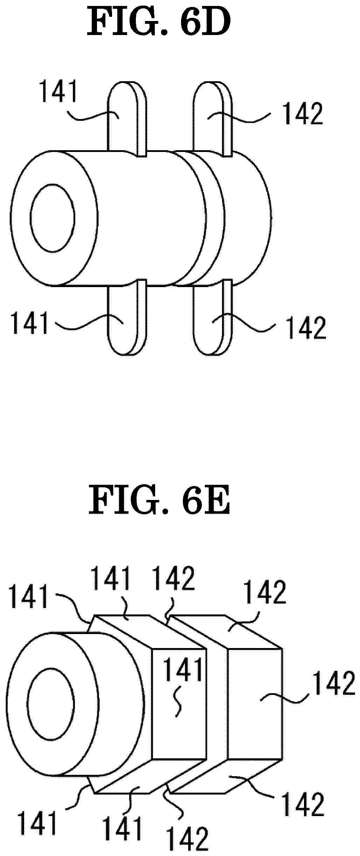

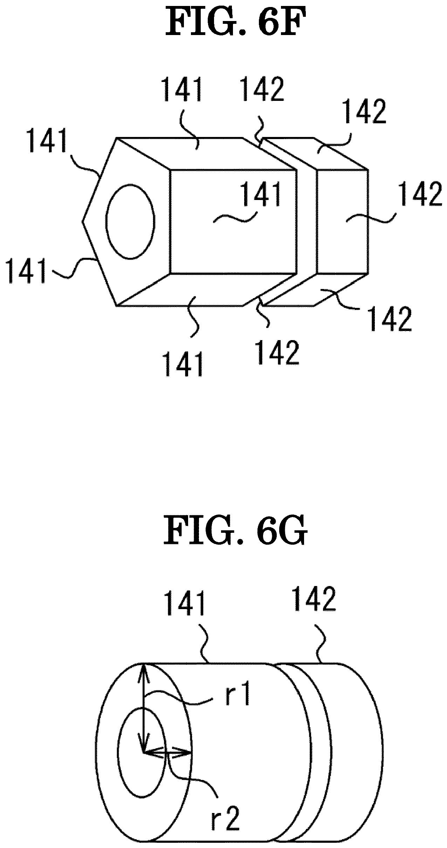

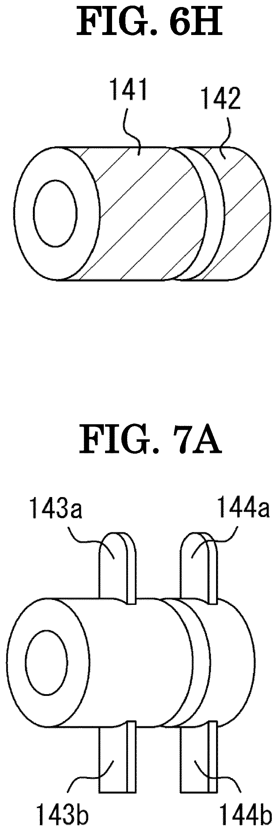

The holding shape portion 140 is not limited to the above-described configuration. For example, as illustrated in FIG. 6A, the holding shape portion 140 may be cut portions provided at two sections. As a matter of course, as illustrated in FIG. 6B, the holding shape portion 140 may be cut portions provided at four sections. In addition, as illustrated in FIG. 6C, recessed portions (grooves or steps) may be provided as the holding shape portion 140. In addition, as illustrated in FIG. 6D, protruded portions (protrusions or steps) may be provided. In addition, as illustrated in FIG. 6E, the holding shape portion 140 may have a pentagonal shape. As a matter of course, the shape is not limited to the pentagonal shape and the hexagonal shape, and other polygonal shapes may be used. In addition, as illustrated in FIG. 6F, the holding shape portion 140 may be integrally formed with the mass member 130. For example, the holding shape portion 140 may be integrally formed with the mass member 130 to form the mass member 130 in a polygonal shape as a whole. In addition, as illustrated in FIG. 6G, the holding shape portion 140 may be integrally formed with the mass member 130 to form the mass member 130 in an elliptical shape having a major diameter r1 and a minor diameter r2 whose lengths are different from each other. As illustrated in FIG. 6H, as the holding shape portion 140, the mass member 130 may be subjected to roughing to increase a friction coefficient.

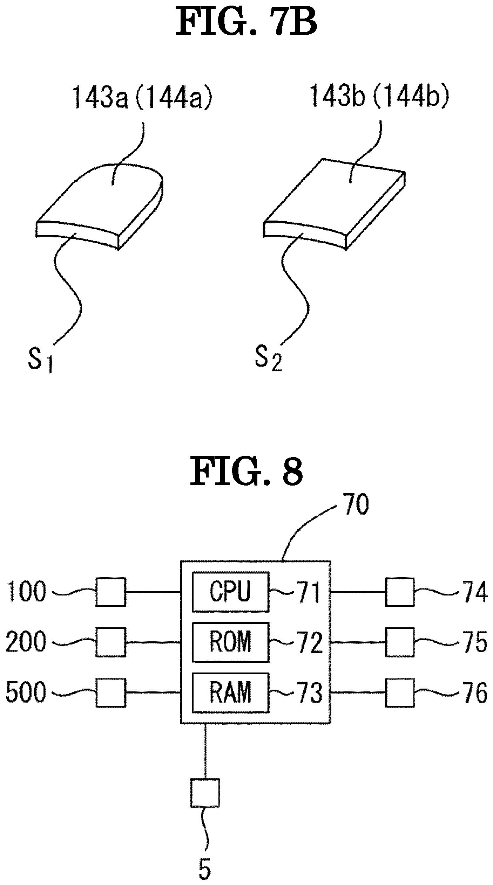

The holding shape portions 140 disposed at symmetrical positions such as mirror-symmetrical positions or rotationally symmetrical positions may respectively have different shapes. For example, as illustrated in FIG. 7A, a holding shape portion 143a and a holding shape portion 143b which have a symmetrical positional relationship in the sonotrode 131 may have different shapes. In addition, a holding shape portion 144a and a holding shape portion 144b which have a symmetrically positional relationship in the back mass 132 may have different shapes. In this case, it is preferable that areas of joint surfaces between the mass member 130 and the holding shape portion 140 are the same as each other. For example, as illustrated in FIG. 7B, it is preferable that an area of a joint surface S1 between the holding shape portion 143a (or 144a) and the sonotrode 131 (or the back mass 132) which have a symmetrical positional relationship and an area of a joint surface S2 between the holding shape portion 143b (or 144b) and the sonotrode 131 (or the back mass 132) are the same as each other. In this manner, the holding shape portion 140 can be prevented from hindering generation of the ultrasonic wave as much as possible.

When the opening portion 101 is provided in the Langevin-type transducer as in the present example, the holding shape portion 140 may be provided inside the opening portion 101. In this case, when the Langevin-type transducer is assembled, a jig attached to the inside of the opening portion 101 is used.

When the holding shape portions 140 are provided at positions which are mirror-symmetrical, the ultrasonic actuator 100 may be attached to the ultrasonic ophthalmic tonometer 1 so that a direction of a symmetry axis is an upward-downward direction of the ultrasonic ophthalmic tonometer 1. In this manner, measurement can be performed under the same condition when a right eye is measured and when a left eye is measured.

The ultrasonic actuator 100 may include an insulation member 170. For example, the insulation member 170 prevents the electrode 120 or the ultrasonic element 110 from coming into contact with the tightening member 160. For example, the insulation member 170 is disposed between the electrode 120 and the tightening member 160. For example, the insulation member 170 has a sleeve shape.

### <Optical Unit>

For example, the optical unit 200 observes or measures the subject eye (refer to FIG. 2). For example, the optical unit 200 includes an objective system 210, a front surface imaging system 220, a fixation target projection system 230, an index projection system 250, a deformation detection system 260, a corneal thickness measurement system 270, a working distance detection system 280, and a cross section imaging system 290, a dichroic mirror 201, a beam splitter 202, a beam splitter 203, and a beam splitter 204.

For example, the objective system 210 is an optical system for fetching light from the outside of the measurement unit 3 to the optical unit 200 or irradiating the outside of the measurement unit 3 with the light from the optical unit 200. For example, the objective system 210 includes an optical element. The objective system 210 may include an optical element (objective lens or relay lens).

An illumination optical system 240 illuminates the subject eye. For example, the illumination optical system 240 illuminates the subject eye with infrared light. For example, the illumination optical system 240 includes an illumination light source 241. For example, the illumination light source 241 is diagonally disposed in front of the subject eye. For example, the illumination light source 241 emits the infrared light. The illumination optical system 240 may include a plurality of the illumination light sources 241.

For example, the front surface imaging system 220 captures an observation image of the subject eye. For example, the front surface imaging system 220 captures an image of an anterior chamber of the subject eye. For example, the front surface imaging system 220 includes a light receiving lens 221 and a light receiving element 222. For example, the front surface imaging system 220 receives the light emitted from the illumination light source 241 and reflected by the subject eye. For example, the front surface imaging system 220 receives a reflection light flux from the subject eye centered on the optical axis O1. For example, reflection light from the subject eye passes through the opening portion 101 of the ultrasonic actuator 100, and is received by the light receiving element 222 via the objective system 210 and the light receiving lens 221.

For example, the fixation target projection system 230 projects a fixation target onto the subject eye. For example, the fixation target projection system 230 includes a target light source 231, a diaphragm 232, a light projection lens 233, and a diaphragm 234. The light from the target light source 231 passes through the diaphragm 232, the light projection lens 233, and the diaphragm 232 along the optical axis O2, and is reflected by the dichroic mirror 201. For example, the dichroic mirror 201 causes the optical axis O2 of the fixation target projection system 230 to be coaxial with the optical axis O1. The light from the target light source 231 reflected by the dichroic mirror 201 passes through the objective system 210 along the optical axis O1, and is emitted to irradiate the subject eye. A target of the fixation target projection system 230 is fixed by a subject. Accordingly, a line of sight of the subject is stabilized.

For example, the index projection system 250 projects an index onto the subject eye. The index projection system 250 projects an index for XY-alignment on the subject eye. For example, the index projection system 250 includes an index light source (for example, an infrared light source) 251, a diaphragm 252, and a light projection lens 253. The light from the index light source 251 passes through the diaphragm 252 and the light projection lens 253 along the optical axis O3, and is reflected by the beam splitter 202. For example, the beam splitter 202 causes the optical axis O3 of the index projection system 250 to be coaxial with the optical axis O1. The light of the index light source 251 which is reflected by the beam splitter 202 passes through the objective system 210 along the optical axis O1, and is emitted to irradiate the subject eye. The light of the index light source 251 which is emitted to irradiate the subject eye is reflected by the subject eye, passes through the objective system 210 and the light receiving lens 221 again along the optical axis O1, and is received by the light receiving element 222. For example, the index received by the light receiving element is used for XY-alignment. In this case, for example, the index projection system 250 and the front surface imaging system 220 function as XY-alignment detection means.

For example, the deformation detection system 260 detects a deformation state of a cornea of the subject eye. For example, the deformation detection system 260 includes a light receiving lens 261, a diaphragm 262, and a light receiving element 263. For example, the deformation detection system 260 may detect the deformation state of the cornea, based on corneal reflection light received by the light receiving element 263. For example, the deformation detection system 260 may detect the deformation of the cornea when the light receiving element 263 receives the light reflected by the cornea of the subject eye after the light is emitted from the index light source 251. For example, the corneal reflection light passes through the objective system 210 along the optical axis O1, and is reflected by the beam splitter 202 and the beam splitter 203. Then, the corneal reflection light passes through the light receiving lens 261 and the diaphragm 262 along the optical axis O4, and is received by the light receiving element 263.

For example, the deformation detection system 260 may detect the deformation state of the cornea, based on a magnitude of a light receiving signal of the light receiving element 236. For example, the deformation detection system 260 may detect that the cornea is in an applanation state when the receiving amount of the light received by the light receiving element 236 is maximized. In this case, for example, the deformation detection system 260 is set so that the receiving amount of the light is maximized when the cornea of the subject eye is in the applanation state.

For example, the corneal thickness measurement system 270 measures a corneal thickness of the subject eye. For example, the corneal thickness measurement system 270 may include a light source 271, a light projection lens 272, a diaphragm 273, a light receiving lens 274, and a light receiving element 275. For example, the light from the light source 271 passes through the light projection lens 272 and the diaphragm 273 along the optical axis O5, and is emitted to irradiate the subject eye. Then, the reflection light reflected by the subject eye is collected by the light receiving lens 274 along the optical axis O6, and is received by the light receiving element 275.

For example, the working distance detection system 280 detects an alignment state in a Z-direction. For example, the working distance detection system 280 includes a light receiving element 281. For example, the working distance detection system 280 may detect the alignment state in the Z-direction by detecting the reflection light from the cornea. For example, the working distance detection system 280 may receive the reflection light reflected by the cornea of the subject eye after the light is emitted from the light source 271. In this case, for example, the working distance detection system 280 may receive a bright spot formed by the light reflected by the cornea of the subject eye after the light is emitted from the light source 271. In this way, the light source 271 may also serve as a light source for detecting a working distance. For example, the light emitted from the light source 271 and reflected by the cornea is reflected by the beam splitter 204 along the optical axis O6, and is received by the light receiving element 281.

### <Detection Unit>

For example, a detection unit 500 detects an output of the ultrasonic actuator 100. For example, the detection unit 500 is a sensor such as an ultrasonic sensor, a displacement sensor, and a pressure sensor. The ultrasonic sensor detects the ultrasonic wave generated from the ultrasonic actuator 100. The displacement sensor detects displacement of the ultrasonic actuator 100. The displacement sensor may detect an oscillation when the ultrasonic actuator 100 generates the ultrasonic wave by continuously detecting the displacement.

As illustrated in FIG. 2, the detection unit 500 is disposed outside an irradiation path A of the ultrasonic wave. For example, the irradiation path A is a region connecting a front surface F of the ultrasonic actuator 100 and an irradiation target Ti of the ultrasonic wave. For example, the detection unit 500 is disposed on a lateral side or on a rear side of the ultrasonic actuator 100. When the detection unit 500 is disposed on the lateral side as in the present example, the subject eye is easily observed by the front surface imaging system 220. When the ultrasonic sensor is used as the detection unit 500, the detection unit 500 detects the ultrasonic wave leaking from the lateral side or the rear side of the ultrasonic actuator 100. When the displacement sensor is used as the detection unit 500, the detection unit 500 detects the displacement of the ultrasonic actuator 100 from the lateral side or the rear side of the ultrasonic actuator 100. For example, the displacement sensor irradiates the ultrasonic actuator 100 with a laser beam, and detects the displacement of the ultrasonic actuator 100, based on the reflected laser beam. A detection signal detected by the detection unit 500 is transmitted to a control unit.

### <Control Unit>

A configuration of a control system will be described with reference to FIG. 8. For example, a control unit 70 controls the whole device, and performs arithmetic processing for measurement values. For example, the control unit 70 is realized by a general central processing unit (CPU) 71, a ROM 72, or a RAM 73. The ROM 72 stores various program s or initial values for controlling an operation of the ultrasonic ophthalmic tonometer 1. The RAM 73 temporarily stores various types of information. The control unit 70 may be configured to include a single control unit or a plurality of control units (that is, a plurality of processors). For example, the control unit 70 may be connected to the drive unit 5, the storage unit 74, the display unit 75, the operation unit 76, the ultrasonic actuator 100, the optical unit 200, or the detection unit 500.

The storage unit 74 is a non-transitory storage medium that can hold stored contents even when power supply is cut off. For example, a hard disk drive, a flash ROM, or a detachable USB memory can be used as the storage unit 74.

For example, the display unit 75 displays a measurement result of the subject eye. The display unit 75 may have a touch panel function.

The operation unit 76 receives various operation instructions from an examiner. The operation unit 76 outputs an operation signal corresponding to the input operation instruction to the control unit 70. For example, as the operation unit 76, at least one user interface such as a touch panel, a mouse, a joystick, and a keyboard may be used. When the display unit 75 is the touch panel, the display unit 75 may function as the operation unit 76.

### <Measurement Operation>

A control operation of the device having the above-described configuration will be described. First, the control unit 70 aligns the ultrasonic ophthalmic tonometer 1 with the subject eye of the subject whose face is supported by the face support unit 4. For example, the control unit 70 detects a bright spot formed by the index projection system 250 from a front image acquired by the light receiving element 222, and drives the drive unit 5 so that a position of the bright spot is a predetermined position. As a matter of course, the examiner may manually perform the alignment with the subject eye by using the operation unit 76 while observing the display unit 75. When the drive unit 5 is driven, the control unit 70 determines whether or not the alignment is proper, based on whether or not the position of the bright spot in the image of the anterior chamber is a predetermined position.

After the alignment with the subject eye E is completed, the control unit 70 causes the corneal thickness measurement system 270 to measure the corneal thickness. For example, the control unit 70 calculates the corneal thickness, based on a light receiving signal received by the light receiving element 275. For example, based on the light receiving signal, the control unit 70 may obtain the corneal thickness from a positional relationship between a peak value of the reflection light on a front surface of the cornea and a peak value of the reflection light on a rear surface of the cornea. For example, the control unit 70 stores the obtained corneal thickness in a storage unit 74.

Subsequently, the control unit 70 measures an intraocular pressure of the subject eye with using the ultrasonic actuator 100. For example, the control unit 70 applies a voltage burst signal to the ultrasonic element 110, and irradiates the subject eye E with the ultrasonic wave. When an acoustic radiation pressure is generated in the cornea of the subject eye by the ultrasonic wave emitted for irradiation from the ultrasonic actuator 100, the cornea is deformed. The control unit 70 causes the deformation detection system 260 to detect the deformation state of the cornea. For example, the control unit 70 detects that the cornea is deformed into a predetermined shape (applanation state or flattened state), based on the light receiving signal of the light receiving element 263. The acoustic radiation pressure gradually increases, and the cornea is flattened (applanation state). In this case, a signal of the deformation detection system 260 is maximized, and the control unit 70 determines that the cornea is in the applanation state.

When the acoustic radiation pressure further increases, the cornea is depressed. In this case, the light receiving signal of the deformation detection system 260 is weakened. The control unit 70 gradually weakens and stops the irradiation of the ultrasonic wave. Then, the cornea returns from the depressed state to the applanation state. In this case, the light receiving signal is maximized again. Thereafter, as the cornea returns to the original shape, the light receiving signal of the deformation detection system 260 is weakened.

For example, the control unit 70 calculates the intraocular pressure of the subject eye, based on the acoustic radiation pressure when the cornea of the subject eye is deformed into a predetermined shape. The acoustic radiation pressure applied to the subject eye correlates with an irradiation time of the ultrasonic wave, and increases as the irradiation time of the ultrasonic wave is lengthened. Therefore, the control unit 70 obtains the acoustic radiation pressure when the cornea is deformed into a predetermined shape, based on the irradiation time of the ultrasonic wave. A relationship between the acoustic radiation pressure when the cornea is deformed into the predetermined shape and the intraocular pressure of the subject eye is obtained in advance by an experiment, and is stored in the storage unit 74. The control unit 70 determines the intraocular pressure of the subject eye, based on the acoustic radiation pressure when the cornea is deformed into the predetermined shape and the relationship stored in the storage unit 74.

As described above, the ultrasonic ophthalmic tonometer of the present example has the holding shape portion 140 for preventing the Langevin-type transducer from rotating with respect to the jig when the Langevin-type transducer is assembled. In addition, the holding shape portions 140 are disposed at positions of a mass member 130 (sonotrode 131 or back mass 132) which are rotationally symmetrical around the sound axis Q1, positions which are mirror-symmetrical with respect to the axis orthogonal to the sound axis Q1, or at both the positions. In this manner, the Langevin-type transducer can be assembled with a proper torque, and an adverse effect on the output of the ultrasonic wave can be suppressed.

A method for calculating the intraocular pressure is not limited to the above-described example, and various methods may be used. For example, the control unit 70 may obtain the deformation amount of the cornea by using the deformation detection system 260, and may obtain the intraocular pressure by multiplying the deformation amount by a conversion coefficient. In addition, for example, the control unit 70 may correct an intraocular pressure value calculated according to the corneal thickness of the subject eye.

The control unit 70 may measure the intraocular pressure, based on the ultrasonic wave reflected by the subject eye. For example, the intraocular pressure may be measured, based on a characteristic change in the ultrasonic wave reflected by the subject eye, or the deformation amount of the cornea may be acquired from the ultrasonic wave reflected by the subject eye to measure the intraocular pressure, based on the deformation amount.

### Reference Signs List

1 Ultrasonic ophthalmic tonometer
2 Base
3 Measurement unit
4 Face support unit
5 Drive unit
70 Control unit
75 Display unit
76 Operation unit
100 Ultrasonic actuator
101 Opening portion
140 Holding shape portion
200 Optical unit

## Claims

1. An ultrasonic ophthalmic tonometer that measures an intraocular pressure of a subject eye with using an ultrasonic wave, the ultrasonic ophthalmic tonometer comprising:
a Langevin-type transducer that irradiates the subject eye with the ultrasonic wave; and
a holding shape portion for preventing the Langevin-type transducer from rotating with respect to a jig when the Langevin-type transducer is assembled,
wherein the holding shape portion is provided in at least one of positions which are mirror-symmetrical with respect to a plane perpendicular to a sound axis of the Langevin-type transducer and positions which are rotationally symmetrical around the sound axis.

2. The ultrasonic ophthalmic tonometer according to claim 1,
wherein the Langevin-type transducer includes a sonotrode, a back mass, and an ultrasonic element sandwiched between the sonotrode and the back mass, and
the holding shape portion is provided in at least one of the sonotrode and the back mass.

3. The ultrasonic ophthalmic tonometer according to claim 1 or 2,
wherein the holding shape portion is at least one of a cut portion, a recessed portion, a protruded portion, and a rough surface portion.

4. The ultrasonic ophthalmic tonometer according to claim 2,
wherein the holding shape portion is integrally formed with the sonotrode or the back mass to form the sonotrode or the back mass in a polygonal shape or an elliptical shape.

5. The ultrasonic ophthalmic tonometer according to any one of claims 1 to 4,
wherein the Langevin-type transducer has an opening portion.
